# EUROPEAN PATENT APPLICATION

(11) **EP 4 674 462 A1**
(43) Date of publication of application: **07.01.2026**
(21) Application number: 23939253.3
(22) Date of filing: 30.10.2023
(51) Int. Cl.: A61M 39/06, A61M 25/00, A61B 17/00

(54) **VASCULAR SHEATH HEMOSTASIS VALVE AND MEDICAL INSTRUMENT**

(30) Priority: 30.05.2023 CN 202310630685
(71) Applicant: Guanqiao Medical Co., Ltd, Beijing 101500 (CN)
(72) Inventor: XU, Dongyue, Beijing 101500 (CN); MA, Lijin, Beijing 101500 (CN)
(74) Representative: Ström & Gulliksson AB
(86) International application number: PCT/CN2023/127908
(87) International publication number: WO 2024/244282

(57) **Abstract**

The present disclosure relates to a vascular sheath hemostasis valve and a medical instrument. The vascular sheath hemostasis valve is used for allowing a vascular sheath to be inserted and to pass through, and comprises a head end, a sleeve and a torsion assembly which are sequentially arranged; a hemostatic film tube is arranged in the sleeve; the head end is fixedly connected to the sleeve; the distal end of the hemostatic film tube is fixed to the joint of the sleeve and the head end; the proximal end of the hemostatic film tube is connected to the torsion assembly; the torsion assembly can rotate relative to the sleeve and is used for driving the hemostatic film tube to tighten and wrap or loosen and release the vascular sheath. According to the vascular sheath hemostasis valve in the present disclosure, an inner sheath tube or a catheter-type instrument of the vascular sheath can smoothly pass through the hemostasis valve, large hoop pressure is generated on the instrument, and the hemostatic effect is guaranteed.

## Description

### CROSS REFERENCE TO RELATED APPLICATION

The present disclosure claims the priority to the Chinese patent application with the filing No. 2023106306857 filed with the Chinese Patent Office on May 30, 2023, and entitled "VASCULAR SHEATH HEMOSTASIS VALVE AND MEDICAL INSTRUMENT", which is incorporated herein by reference in entirety.

### TECHNICAL FIELD

The present disclosure belongs to the technical field of medical instruments, and in particular, relates to a vascular sheath hemostatic valve and a medical instrument.

### BACKGROUND

Vascular sheaths are medical instruments that are mainly used for interventional treatment or as auxiliary instruments during surgical treatments. During surgery, a vascular sheath can be inserted into a vascular system through percutaneous puncture to protect the blood vessels. A hemostatic valve is usually provided at the proximal end of the vascular sheath to reduce blood loss when the vascular sheath passes through a catheter or guidewire during surgery.

### SUMMARY

The present disclosure provides a vascular sheath hemostatic valve for allowing vascular sheath to be inserted and to pass through, the vascular sheath hemostatic valve includes a head end, a sleeve and a torsion assembly sequentially arranged, a hemostatic film tube is provided in the sleeve, and the head end is fixedly connected to the sleeve;
a distal end of the hemostatic film tube is fixed to a joint of the sleeve and the head end, and a proximal end of the hemostatic film tube is connected on the torsion assembly, and the torsion assembly is rotatable relative to the sleeve to drive the hemostatic film tube to tighten and wrap or loosen and release the vascular sheath.

Optionally, a distal end of the sleeve is inserted into a proximal end of the head end, and the distal end of the sleeve and the proximal end of the head end are in a detachable fixed connection.

Optionally, the detachable fixed connection is selected from one of snap-fit fixation, plug-in fixation and screw-on fixation.

Optionally, the distal end of the sleeve is inserted into the proximal end of the head end, a buckle is provided on an outer side wall of the sleeve, and a slot is provided on a side wall of the head end, and the sleeve is snap-fitted and fixed to the head end through a cooperation of the buckle and the slot.

Optionally, an annular step surface is provided inside the head end for abutting against an end surface of the distal end of the sleeve; sealing members are provided at the joint of the sleeve and the head end.

Optionally, the sealing members are provided on the annular step surface or the end surface of the distal end of the sleeve; and/or
the sealing members are provided on the outer side wall of the sleeve or an inner side wall of the head end.

Optionally, the sealing members are sealing rings.

Optionally, the annular step surface is provided inside the head end for the end surface of the sleeve to abut against; sealing rings are provided both on the annular step surface and on the outer side wall of the sleeve where the sleeve is inserted into the head end; the distal end of the hemostatic film tube protrudes from the distal end of the sleeve and is press-fitted and fixed between the head end and the sleeve after being folded.

Optionally, the torsion assembly includes a torsion handle and a connector arranged inside the torsion handle, and the connector is fixedly connected to the torsion handle.

Optionally, the fixed connection is a detachable fixed connection; the detachable fixed connection is selected from one of snap-fit fixation, plug-in fixation or screw-on fixation.

Optionally, the torsion assembly includes the torsion handle and the connector arranged inside the torsion handle, a buckle is provided on the connector, and a slot is provided on a side of the torsion handle, and the connector is snap-fitted and fixed to the torsion handle through a cooperation of the buckle and the slot.

Optionally, the distal end of the connector is inserted into the proximal end of the sleeve, an annular groove is provided at a proximal opening of the sleeve, and the connector includes an annular boss located at the distal end, and the annular boss is fitted and accommodated in the annular groove.

Optionally, a proximal end of the connector is inserted onto the torsion handle, and an end surface of the proximal end of the connector abuts against an inner side wall surface or an inner side end surface of the torsion handle; sealing members are provided at the joint of the torsion handle and the connector.

Optionally, the sealing members are provided on an end surface of the proximal end of the connector or an inner side wall surface or an inner side end surface of the torsion handle, and/or
on an inner side wall of the proximal end of the connector or an outer side wall of the torsion handle.

Optionally, the sealing members are sealing rings.

Optionally, the proximal end of the connector is inserted onto the torsion handle, an end surface of the connector abuts against an inner side wall surface of the torsion handle, and sealing rings are provided both on an abutment portion between the torsion handle and the connector, as well as on an outer side wall of the proximal end of the connector, and a proximal end of the hemostatic film tube protrudes from the proximal end of the connector and is press-fitted and fixed between the connector and the torsion handle after being folded.

Optionally, the hemostatic film tube includes an elastic film tube, and the torsion assembly is limited and restrained on the sleeve by an axial contraction elastic force of the elastic film tube.

Optionally, the hemostatic film tube includes a high molecular polymer.

Optionally, the high molecular polymer is selected from one or more of silicone resin, thermoplastic polyurethane elastomer (TPU), expanded polytetrafluoroethylene (ePTFE), polyisocyanate or rubber.

Optionally, an arc-shaped buckle is provided on an annular inner wall of the torsion handle, and a locking bevel tooth is provided on an annular outer wall of the sleeve, and the arc-shaped buckle is snap-fitted with the locking bevel tooth.

Optionally, a limiting boss is provided on the torsion handle, the limiting boss is protrudingly provided on the end surface of the torsion handle, and a limiting block capable of preventing the limiting boss from excessive rotation is provided on the sleeve.

The present disclosure also provides a medical instrument, the medical instrument includes the vascular sheath hemostatic valve as mentioned above and a vascular sheath, the vascular sheath includes an inner sheath joint, the inner sheath joint is inserted onto the torsion assembly, a locking buckle is provided on the torsion assembly, and a locking groove that cooperates with the locking buckle is provided on the inner sheath joint.

Optionally, the torsion assembly includes a torsion handle and a connector arranged inside the torsion handle, the inner sheath joint is inserted onto the torsion handle, a locking buckle is provided on the torsion handle, and a locking groove that cooperates with the locking buckle is provided on the inner sheath joint.

### BRIEF DESCRIPTION OF DRAWINGS

In order to more clearly illustrate the technical solutions of the embodiments of the present disclosure, the following briefly introduces the drawings required for use in the embodiments. It should be understood that the following drawings only illustrate embodiments of the present disclosure and therefore should not be regarded as limiting the scope. For those ordinarily skilled in the art, other relevant drawings can be obtained based on these drawings without any inventive effort. It should be understood that for those ordinarily skilled in the art, other relevant drawings can be obtained based on these drawings without any inventive effort.
Figure 1 is a schematic structural diagram of a vascular sheath hemostatic valve provided in an embodiment of the present disclosure;
Figure 2 is a schematic structural diagram of a hemostatic film tube in the vascular sheath hemostatic valve in Figure 1 under different tightening conditions;
Figure 3 is a schematic structural diagram of a vascular sheath hemostatic valve provided in an embodiment of the present disclosure from another perspective;
Figure 4 is an enlarged view of a partial structure of the vascular sheath hemostatic valve in Figure 3;
Figure 5 is a schematic diagram of a state in which a hemostatic film tube is tightened and wrapped around a vascular sheath in one embodiment of the present disclosure;
Figure 6 is a schematic diagram of a state in which the hemostatic film tube of the vascular sheath hemostatic valve is completely tightened without an introduced item in one embodiment of the present disclosure;
Figure 7 is a schematic diagram of a state of an inner sheath joint and the torsion handle in a locking process in one embodiment of the present disclosure;
Figure 8 is a schematic structural diagram of a vascular sheath in cooperation with a hemostatic valve in one embodiment of the present disclosure;
Figure 9 is a schematic structural diagram of a torsion assembly taking from a section A-A in the hemostatic valve of Figure 8.

### Reference numerals:

1-head end; 11-annular step surface;
2-sleeve; 21-annular groove; 22-locking bevel tooth; 23-limiting block;
3-torsion assembly; 31-torsion handle; 31a-arc-shaped buckle; 31b-limiting boss; 31clocking buckle; 32-connector; 32a-annular boss;
4-hemostatic film tube;
5-vascular sheath; 51-inner sheath joint; 52-inner sheath tube; 53-locking groove; 54-outer sheath tube;
10-buckle;
20-slot;
30-sealing ring.

### DETAILED DESCRIPTION OF EMBODIMENTS

In order to make the objective, technical solutions and advantages of the embodiments of the present disclosure clearer, the technical solutions in the embodiments of the present disclosure will be described clearly and completely below with reference to the drawings in the embodiments of the present disclosure. Obviously, the described embodiments are only part of the embodiments of the present disclosure, rather than all the embodiments. The components of the embodiments of the present disclosure generally described and illustrated in the figures herein could be arranged and designed in a wide variety of different configurations.

In the description of the present disclosure, it should be noted that the terms "inside" and "outside" etc. indicate orientations or positional relationships are based on the orientations or positional relationships shown in the drawings, or are the orientations or positional relationships in which the disclosed product is usually placed when in use. These are only for the convenience of describing the present disclosure and simplifying the description, and do not indicate or imply that the device or element referred to must have a specific orientation, be constructed and operated in a specific orientation. Therefore, they should not be understood as limitations on the present disclosure. Furthermore, the terms "first", "second", etc. are merely used for distinguishing descriptions and should not be understood as indicating or implying relative importance.

In the description of the present disclosure, it should also be noted that, unless otherwise explicitly specified and limited, the terms "provided" and "connection" should be understood in a broad sense. For example, it can be a fixed connection, a detachable connection, or an integral connection; it can be a direct connection, or an indirect connection through an intermediate medium, or it can be an internal communication between two elements. For those ordinarily skilled in the art, the specific meanings of the above terms in this disclosure can be understood according to specific circumstances.

In the description of the present disclosure, it should also be noted that, unless otherwise explicitly specified and limited, the terms "proximal end" and "distal end" refer to the relative orientation, relative position and direction of elements or actions relative to each other from a perspective of an operator using the medical instrument. Although "proximal end" and "distal end" are not restrictive, the "proximal end" generally refers to an end of the medical instrument that is close to the operator during normal operation, and the "distal end" generally refers to an end away from the operator, such as the end that first enters a body of patient or the end that is close to the patient during normal operation. Furthermore, the term "or" in the above embodiments is generally used in a sense including "and/or", unless otherwise explicitly stated. In the above embodiment, "two ends" refer to the proximal end and the distal end.

Common vascular sheath hemostatic valves on the market are classified into two types: twist-type hemostatic valves and insertion-extraction type hemostatic valves. The twist-type hemostatic valve achieves hemostatic effect by twisting a handle to make the handle compress an annular silicone pad and deform the silicone pad, thereby reducing or blocking a pathway. It is difficult to control the compression degree of the hemostatic valve when the hemostatic valve is twisted. Excessive force will cause the silicone pad to over-press the catheter, causing the catheter to deform, while insufficient force will reduce the hemostatic effect. At the same time, when there is a catheter that needs to be pushed in the hemostatic valve, it is difficult for the twist-type hemostatic valve to achieve the effect of both smoothly pushing the catheter and effectively stopping bleeding.

The insertion-extraction type hemostatic valve stops bleeding through a circular silicone sheet with a cross incision in the middle. When in use, press a handle, and a cylindrical rod with a passage in the handle passes through the cross incision in the middle of the circular silicone sheet to open the passage. Retract the handle, and the cylindrical rod exits the circular silicone sheet so that the cross incision of the silicone sheet is closed to achieve hemostasis. This hemostatic method cannot withstand a relatively large pressure, and the hemostatic effect will vary depending on the diameter of the inserted catheter. The larger the diameter, the smaller the pressure bearing capacity and a worse the hemostatic effect.

The vascular sheath hemostatic valve of the present disclosure is mainly used for the hemostasis of an inner sheath tube of a vascular sheath or a catheter-type instrument passing through and inserted into the hemostatic valve. Optionally, the hemostatic effect can be optimized by changing a form of a hemostatic assembly to solve the problems in the prior art that the deformable silicone pad or elastomer cannot control a degree of compression and that there are differences in the hemostatic effects of catheters of different diameters.

Optionally, by changing the existing silicone pad, elastomer or silicone sheet into a form of a hemostatic film tube, an original compression hemostasis is changed to a form of tightening and wrapping an outside of the catheter, and by controlling a rotation angle of the hemostatic film tube, the applied tightening and wrapping force is controlled, so that a compression force on the catheter can be controlled.

Moreover, through the form of tightening and wrapping, the limitation of different pressure-bearing capacity of catheters of different diameters is broken through. Different tightening and wrapping forces can be applied by controlling a rotation amount of the hemostatic film tube, so that it is suitable for instruments of different diameters and ensures a good hemostatic effect on it.

The hemostatic film tube provided in the present disclosure can wrap the vascular sheath passing through and inserted into the hemostatic valve; in combination with fixing the distal end of the hemostatic film tube at a joint of the sleeve and the head end, and connecting the proximal end of the hemostatic film tube to a torsion assembly, and enabling the torsion assembly to rotate relative to a sleeve, a state of the hemostatic film tube can be adjusted; by rotating the torsion assembly forward or backward, the hemostatic film tube can be driven by the torsion assembly to tighten and wrap or loosen and release the vascular sheath.

The vascular sheath hemostatic valve and medical instrument provided by the present disclosure can allow the inner sheath tube of the vascular sheath or catheter-type instruments to pass through the hemostatic valve smoothly, and generate a large hoop pressure on the instrument to ensure the hemostatic effect. An embodiment of the present disclosure provides a vascular sheath hemostatic valve, which uses a tightening and wrapping method through a hemostatic film tube to effectively solve the problem of excessive pressure and grip of the vascular sheath or catheter-type instruments by silicone pads or elastomers in the prior art. At the same time, the tightening and wrapping hemostatic method is more suitable for vascular sheaths or catheter-type instruments of different calibers, avoiding differences in hemostatic effects of instruments of different calibers.

Other features and advantages of the present disclosure will be described in detail in the following detailed description.

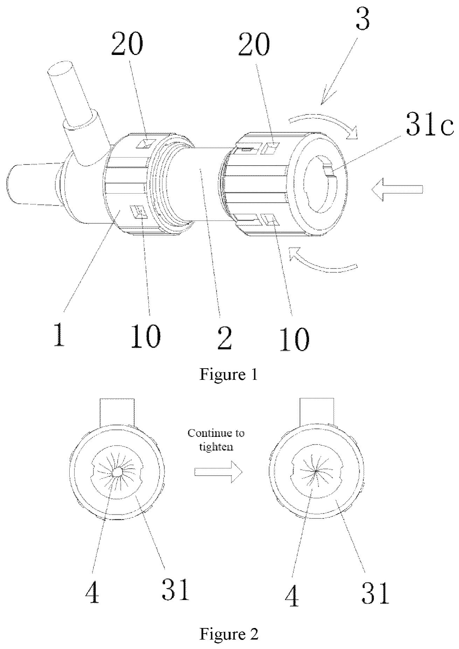

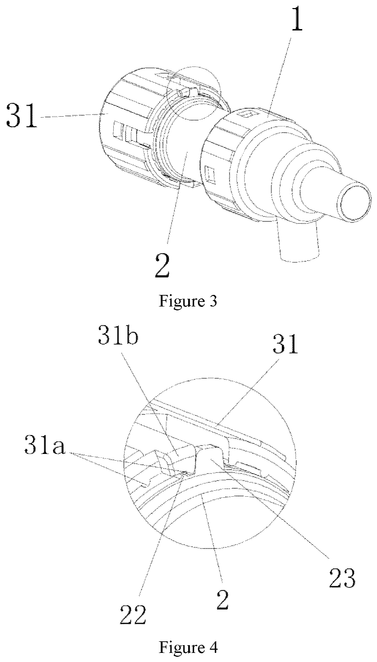

Referring to Figure 1, and in combination with Figures 2 to 4, the vascular sheath hemostatic valve in the present disclosure is mainly used for allowing a vascular sheath 5 to pass through and be inserted, and at the same time, an inner sheath tube 52 of the vascular sheath 5 is wrapped and tightened through a hemostatic film tube 4 to compress the inner sheath tube 52 to achieve the purpose of hemostasis. It should be pointed out that in addition to being used for the vascular sheath 5, the hemostatic valve in the present disclosure can also be used to wrap and tighten the catheter-type products inserted in the hemostatic valve to compress the catheter-type products to achieve the purpose of hemostasis, which is specially explained.

The vascular sheath hemostatic valve of the present disclosure includes a head end 1, a sleeve 2 and a torsion assembly 3 sequentially arranged from distal to proximal. The hemostatic film tube 4 is arranged in the sleeve 2, and is mainly used to tighten and wrap the inner sheath tube 52 of the vascular sheath 5 at the sleeve 2.

The head end 1 is fixedly connected to the sleeve 2. During use, the head end 1 and the sleeve 2 are fixed. By fixing a distal end of the hemostatic film tube 4 at a joint of the sleeve 2 and the head end 1, the distal end of the hemostatic film tube 4 is in a fixed state.

A proximal end of the hemostatic film tube 4 is connected to the torsion assembly 3 and can rotate relative to the fixed sleeve 2 following the torsion assembly 3. The vascular sheath 5 passes through the hemostatic film tube 4 and does not rotate during the hemostatic process. During a rotation of the proximal end of the hemostatic film tube 4, the hemostatic film tube 4 as a whole can be twisted and deformed, thereby tightening and wrapping the vascular sheath 5 in a manner similar to twisting clothes.

Optionally, the torsion assembly 3 can rotate relative to the fixed sleeve 2, and simultaneously drive the proximal end of the hemostatic film tube 4 to rotate synchronously, thereby causing the hemostatic film tube 4 to twist and deform as a whole to tighten and wrap the vascular sheath 5. When the vascular sheath 5 needs to be withdrawn, the torsion assembly 3 is rotated in a opposite direction of the tightening and wrapping rotation, so that the hemostatic film tube 4 releases the wrapping of the vascular sheath 5, thereby loosening and releasing the vascular sheath 5.

Through the hemostatic film tube 4 in the present disclosure, a degree of tightening and wrapping of the hemostatic film tube 4 on the vascular sheath 5 can be controlled by controlling a rotation angle of the torsion assembly 3, so that the catheter can be pushed smoothly while ensuring the hemostatic effect on the vascular sheath 5.

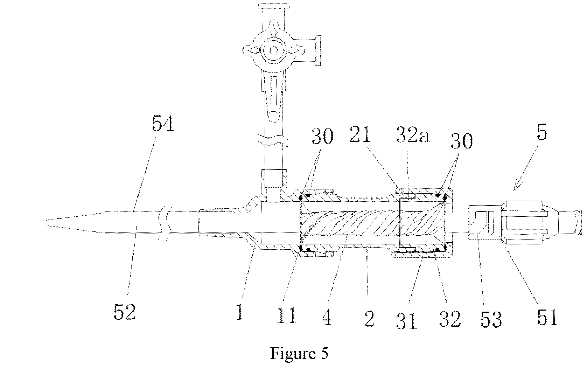

With reference to Figures 5-7, in one of the alternative embodiments, the head end 1 and the sleeve 2 are in a snap-fit fixation relationship, a distal end of the sleeve 2 is inserted into a proximal end of the head end 1, and a buckle 10 is clipped into a slot 20 through the buckle 10 provided on an outer side wall of the sleeve 2 and the slot 20 provided on a side wall of the head end 1, thereby completing the snap-fit fixation between the head end 1 and the sleeve 2.

It should be noted that in the present disclosure, the connection and fixation method between the head end 1 and the sleeve 2 includes but is not limited to a snap-fit structure, and the connection and fixation method can be a plug-in fixation or a screw-on fixation. For example, the snap-fit fixation can be achieved by a buckle and a slot or by an elastic member and a snap-fit member. The plug-in fixation can be achieved by an adapted plug and a socket; the screw-on fixation can be achieved by an adapted internal thread and an external thread.

In an alternative embodiment, the head end 1 and the sleeve 2 are in a plug-in fixation relationship based on the plug and the socket. The distal end of the sleeve 2 is inserted into the proximal end of the head end 1. By arranging the plug on the outer side wall of the sleeve 2, and providing the socket on the side wall of the head end 1, the plug and the socket are mated to complete the plug-in fixation between the head end 1 and the sleeve 2.

In an alternative embodiment, the head end 1 and the sleeve 2 are in a screw-on fixation relationship based on an adapted internal thread and an external thread, and the distal end of the sleeve 2 is screwed into the proximal end of the head end 1. For example, the screw-on fixation between the head end 1 and the sleeve 2 is completed by an external thread provided on the outer side wall of the sleeve 2 and an internal thread provided on the side wall of the head end 1.

In this embodiment, an end surface of the distal end of the sleeve 2 abuts against an annular step surface 11 inside the head end 1. In order to ensure a sealing effect of the connection (abutment portion) between the sleeve 2 and the head end 1, sealing rings 30 are provided both on the annular step surface 11 abutted by the end surface of the distal end of the sleeve 2, and on the outer side wall of the joint of the sleeve 2 with the head end 1. The sealing rings 30 can prevent blood from overflowing at this portion, and can also provide a sealed connection for the distal end of the hemostatic film tube 4.

In other embodiments, in order to ensure the sealing effect of the abutment portion between the sleeve 2 and the head end 1, sealing rings 30 are provided on the end surface of the distal end of the sleeve 2, on the annular step surface 11 abutted by the end surface of the distal end of the sleeve 2, and on the outer side wall of the joint of the sleeve 2 with the head end 1 to prevent blood from overflowing at this portion, and at the same time, it can also provide a sealed connection for the distal end of the hemostatic film tube 4.

In order to press-fit and fix the distal end of the hemostatic film tube 4 at the snap-fitted portion between the head end 1 and the sleeve 2, the distal end of the hemostatic film tube 4 protrudes from the distal end of the sleeve 2 and is press-fitted and fixed between the head end 1 and the sleeve 2 after being folded. Optionally, the distal end of the hemostatic film tube 4 is squeezed and fixed by the two sealing rings 30 mentioned above. Optionally, the distal end of the hemostatic film tube 4 is folded over the sealing ring 30 at the annular step surface 11, and then is press-fitted by means of the sealing ring 30 on the outer side wall of the sleeve 2 where the sleeve 2 is inserted into the head end 1, which can ensure the sealing effect while satisfying the fixing on the hemostatic film tube 4.

In another alternative embodiment, the torsion assembly 3 includes a torsion handle 31 and a connector 32 arranged inside the torsion handle 31, the buckle 10 is provided on the connector 32, and the slot 20 is provided on a side of the torsion handle 31. The connector 32 is snap-fitted and fixed to the torsion handle 31 through a cooperation of the buckle 10 and the slot 20.

In other embodiments, other fixation and connection methods can also be used. For example, a relationship between the torsion handle 31 and the connector 32 is based on the snap-fit fixation relationship between the elastic member and the snap-fit member. The connector 32 is arranged inside the torsion handle 31. By arranging the elastic member on the connector 32, and providing the snap-fit member inside the torsion handle 31, the elastic member and the snap-fit member are mated to complete the snap-fit fixation between the torsion handle 31 and the connector 32.

In other embodiments, the connection and fixation method between the torsion handle 31 and the connector 32 is plug-in fixation. The connector 32 is arranged inside the torsion handle 31. By arranging the plug on the connector 32, and providing the socket inside the torsion handle 31, the plug and the socket are mated to complete the plug-in fixation between the torsion handle 31 and the connector 32.

In an alternative embodiment, the torsion handle 31 and the connector 32 are in a screw-on fixation relationship based on an adapted internal thread and an external thread. The connector 32 is arranged inside the torsion handle 31, and the screw-on fixation between the torsion handle 31 and the connector 32 is completed by the external thread provided on an outer side wall of the connector 32 and the internal thread provided on the side wall of the torsion handle 31.

It should be pointed out that there is no connection relationship between the connector 32 and the sleeve 2, and the connector 32 and the torsion handle 31 form an integral structure after being snap-fitted together, so that the connector 32 can rotate synchronously following the torsion handle 31.

The distal end of the connector 32 is inserted into the proximal end of the sleeve 2. An annular groove 21 is provided at a proximal opening of the sleeve 2. The connector 32 includes an annular boss 32a located at the distal end. The annular boss 32a is fitted and accommodated in the annular groove 21. Optionally, the annular boss 32a is radially recessed in the outer side wall of the connector 32, so that after the connector 32 is inserted into the sleeve 2, the outer side wall of the connector 32 and the side wall of the sleeve 2 are located on the same annular surface, facilitating a free rotation of the connector 32 relative to the sleeve 2.

The connector 32 mainly functions to adapt the proximal end of the hemostatic film tube 4. Optionally, the proximal end of the connector 32 is inserted into the torsion handle 31, and the end surface of the proximal end of the connector 32 abuts against an inner side wall surface or an inner side end surface of the torsion handle 31.

In order to ensure the sealing effect between the connector 32 and the torsion handle 31, sealing rings 30 are provided on the inner side wall surface or the inner side end surface of the abutment portion between the torsion handle 31 and the connector 32, as well as on the outer side wall of the connector 32 where the proximal end of the connector 32 is connected to the torsion handle, so as to ensure the sealing effect between the connector 32 and the torsion handle 31.

In other embodiments, in order to ensure the sealing effect between the connector 32 and the torsion handle 31, sealing rings 30 are provided on the end surface of the proximal end of the connector 32, on the inner side wall surface or the inner side end surface of the torsion handle 31 at the abutment portion with the connector 32, and on the outer side wall of the connector 32 where the proximal end of the connector 32 is connected to the torsion handle.

The proximal end of the hemostatic film tube 4 protrudes from the proximal end of the connector 32 and is press-fitted and fixed to the joint between the connector 32 and the torsion handle 31 after being folded outwardly at the abutment portion between the connector 32 and the torsion handle 31.

Similarly, the proximal end of the hemostatic film tube 4 is squeezed and fixed by the above-mentioned two or more sealing rings 30. Optionally, the proximal end of the hemostatic film tube 4 is press-fitted by the sealing ring 30 on an outer side wall of the proximal end of the connector 32 after being folded over the sealing ring 30 at the abutment portion, thereby ensuring the sealing effect while achieving the fixation of the proximal end of the hemostatic film tube 4.

In this embodiment, an axial limitation of the torsion assembly 3 on the sleeve 2 is achieved by a contraction elastic force of the hemostatic film tube 4 of its own. The hemostatic film tube 4 includes an elastic film tube, and the torsion assembly 3 is limited and restrained at the proximal end of the sleeve 2 by the axial contraction elastic force of the elastic film tube.

In other embodiments, the hemostatic film tube 9 includes an elastic polymer selected from materials such as silicone resin, polyisocyanate, latex, or the like.

In other embodiments, other suitable materials for the hemostatic film tube are selected from expanded polytetrafluoroethylene (ePTFE), silk, polyester fabric or other medical grade materials.

The arrangement of this embodiment simplifies the connection of the torsion assembly 3 on the sleeve 2, and the torsion assembly 3 can be prevented from being detached from the sleeve 2 by the restraint of the contraction elastic force.

The elastic film tube has good elastic deformation effect and can ensure the hemostatic effect on the vascular sheath 5 by changing the shape of the tube when it is tightened and wrapped.

In another alternative embodiment, based on an angle of preventing the torsion handle 31 from rotating freely relative to the sleeve 2, an arc-shaped buckle 31a is provided on the annular inner wall of the torsion handle 31, and a locking bevel tooth 22 is provided on an annular outer wall of the sleeve 2.

When the torsion handle 31 is required to be rotated, the arc-shaped buckle 31a can overcome a limit of the locking bevel teeth 22 under an action of external force and rotate normally.

When rotation is not required, the locking bevel tooth 22 can block and limit the arc-shaped buckle 31a, maintaining a relatively fixed position relationship between the torsion handle 31 and the sleeve 2.

The arc-shaped buckle 31a is snap-fitted with the locking bevel tooth 22, thereby preventing the torsion handle 31 from rotating freely when there is no external force.

The arc-shaped buckle 31a and the locking bevel teeth 22 are evenly distributed on the circumference of the torsion handle 31 and the sleeve 2, respectively, so as to achieve locking at different rotation angles.

Based on the composition of the elastic film tube of the hemostatic film tube 4, in order to prevent it from being damaged or broken due to excessive tightening, and at the same time to facilitate the operator to understand a torsion state of the hemostatic film tube 4, a limiting assembly is provided between the torsion handle 31 and the sleeve 2, so that the relative rotation of the torsion handle 31 and the sleeve 2 is limited to one rotation. Optionally, the limiting assembly includes a limiting boss 31b provided on the torsion handle 31, and a limiting block 23 provided on the outer side wall of the sleeve 2 and capable of preventing the limiting boss 31b from excessively rotating.

Optionally, a limiting boss 31b is protrudingly provided on the end surface of the torsion handle 31, which can reduce the interference between the arc-shaped buckle 31a on the annular inner wall of the torsion handle 31 and the limiting block 23 on the sleeve 2. While ensuring that the torsion handle 31 can rotate normally relative to the sleeve 2, and after reaching a rotation limit position, the limiting block 23 blocks the limiting boss 31b to ensure the limiting effect.

By respectively arranging a locking assembly and the limiting assembly between the torsion handle 31 and the sleeve 2, it is possible to prevent the torsion handle 31 from rotating freely relative to the sleeve 2, and limit a maximum rotation angle of the torsion handle 31, thereby seeking a relative balance between rotation adjustment and rotation limitation, and improving the use effect.

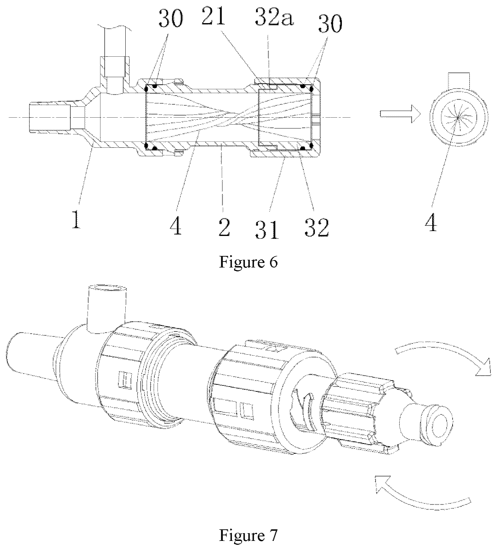

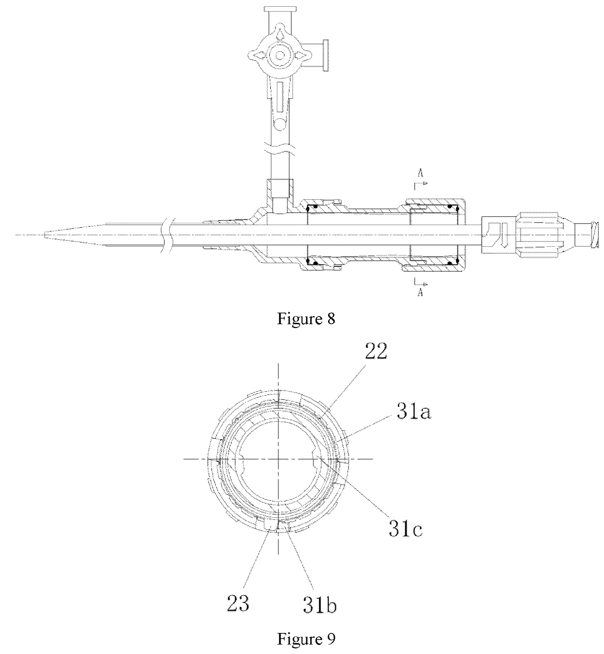

Referring to Figure 7 to Figure 9, and in combination with Figure 5, the present disclosure also provides a medical instrument, including the above-mentioned vascular sheath hemostatic valve and a vascular sheath 5, wherein the vascular sheath 5 includes an inner sheath joint 51 and an inner sheath tube 52 connected to the inner sheath joint 51, the inner sheath tube 52 passes through and is inserted into the hemostatic film tube 4 in the hemostatic valve, the inner sheath joint 51 is inserted on the torsion handle 31, a locking buckle 31c is provided on the torsion handle 31, the inner sheath joint 51 is provided with a locking groove 53 that cooperates with the locking buckle 31c, the locking groove 53 can be optionally a bent counterbore, and the inner sheath joint 51 can enable the locking buckle 31c to enter the locking groove 53 after rotation.

During use, by rotating and screwing the inner sheath joint 51 relative to the torsion handle 31, the inner sheath joint 51 can be locked and assembled on the torsion handle 31 with the cooperation of the locking buckle 31c and the locking groove 53, preventing the inner sheath joint 51 and the inner sheath tube 52 from retreating during the advancement process.

When no inner sheath or catheter-type instrument is introduced into the hemostatic valve, the torsion handle 31 is rotated clockwise, and the hemostatic film tube 4 is tightened to reduce the aperture until it is completely closed to achieve a hemostatic effect. The torsion handle 31 is rotated counterclockwise to twist the hemostatic film open and the passage is restored.

When an inner sheath or catheter-type instrument is introduced into the hemostatic valve, the torsion handle 31 is rotated clockwise, and the hemostatic film tube 4 tighten and wrap around the outer wall of the inner sheath or catheter-type instrument to achieve a hemostatic effect. The torsion handle 31 is rotated counterclockwise to twist the hemostatic film open and the passage is restored.

It should be important to pointed out that, by tightening and wrapping of the hemostatic film tube 4, the vascular sheath hemostatic valve in the present disclosure can prevent the inner sheath tube 52 of the vascular sheath 5 or other catheters from being squeezed and damaged, and can adjust the tightening pressure force, which is suitable for hemostasis of catheter-type instruments with different diameters.

In addition, after an introduced item is tightened and pressed, it can withstand greater pressure on a front end side of the hemostasis site, and can achieve ideal pressure-bearing and hemostatic effects. Even when the vascular sheath 5 or catheter-type products are not introduced, the hemostatic valve can still achieve the purpose of hemostasis by the hemostatic film tube 4 in a tightened state.

An outer sheath tube 54 is also inserted into the distal end of the head end 1. After the inner sheath tube 52 passes through the entire hemostatic valve and the entire outer sheath tube 54, it extends out the distal opening of the outer sheath tube 54. The outer sheath tube 54 includes a long sheath or a short sheath, which can be actually provided according to optional use. A length of the short sheath is about 10 cm, and the length of the long sheath is about 100 cm.

The inner sheath tube 52 includes a tip end at the distal end, which facilitates opening a path in the blood vessel through the tip end. At the same time, the inner sheath tube 52 carries the outer sheath tube 54 into the blood vessel together. When a target position is reached, the inner sheath tube 52 is withdrawn and the outer sheath tube 54 is retained in the blood vessel to form a retention channel in the blood vessel, which facilitates the introduction of other instruments into the blood vessel through the outer sheath tube 54.

### INDUSTRIAL APPLICABILITY

The present disclosure provides a vascular sheath hemostatic valve and a medical instrument. The vascular sheath hemostatic valve in the present disclosure can allow the inner sheath tube of the vascular sheath or catheter-type instruments to pass through the hemostatic valve smoothly, and generate a large hoop pressure on the instrument to ensure the hemostatic effect. It can be widely used in the field of medical instruments and has excellent practical performance.

## Claims

1. A vascular sheath hemostatic valve for allowing vascular sheath to be inserted and to pass through, **characterized in that** the vascular sheath hemostatic valve comprises a head end, a sleeve and a torsion assembly sequentially arranged, wherein a hemostatic film tube is provided in the sleeve, and the head end is fixedly connected to the sleeve;
a distal end of the hemostatic film tube is fixed to a joint of the sleeve and the head end, and a proximal end of the hemostatic film tube is connected on the torsion assembly, and the torsion assembly is rotatable relative to the sleeve to drive the hemostatic film tube to tighten and wrap or loosen and release the vascular sheath.

2. The vascular sheath hemostatic valve according to claim 1, **characterized in that** a distal end of the sleeve is able to be inserted into a proximal end of the head end, and the distal end of the sleeve and the proximal end of the head end are in a detachable fixed connection;
optionally, the detachable fixed connection is selected from one of snap-fit fixation, plug-in fixation and screw-on fixation.

3. The vascular sheath hemostatic valve according to claim 1 or 2, **characterized in that** the distal end of the sleeve is inserted into the proximal end of the head end, a buckle is provided on an outer side wall of the sleeve, and a slot is provided on a side wall of the head end, and the sleeve is snap-fitted and fixed to the head end through a cooperation of the buckle and the slot.

4. The vascular sheath hemostatic valve according to any one of claims 1 to 3, **characterized in that** an annular step surface is provided inside the head end for abutting against an end surface of the distal end of the sleeve;
sealing members are provided at the joint of the sleeve and the head end;
optionally, the sealing members are provided on the annular step surface or the end surface of the distal end of the sleeve; and/or
the sealing members are provided on the outer side wall of the sleeve or an inner side wall of the head end;
optionally, the sealing members are sealing rings.

5. The vascular sheath hemostatic valve according to any one of claims 1 to 4, **characterized in that** the annular step surface is provided inside the head end for the end surface of the sleeve to abut against; sealing rings are provided both on the annular step surface and on the outer side wall of the sleeve where the sleeve is inserted into the head end; the distal end of the hemostatic film tube protrudes from the distal end of the sleeve and is press-fitted and fixed between the head end and the sleeve after being folded.

6. The vascular sheath hemostatic valve according to any one of claims 1 to 5, **characterized in that** the torsion assembly comprises a torsion handle and a connector arranged inside the torsion handle, and the connector is fixedly connected to the torsion handle;
optionally, the fixed connection is a detachable fixed connection; the detachable fixed connection is selected from one of snap-fit fixation, plug-in fixation or screw-on fixation.

7. The vascular sheath hemostatic valve according to claim 6, **characterized in that** the torsion assembly comprises the torsion handle and the connector arranged inside the torsion handle, a buckle is provided on the connector, and a slot is provided on a side of the torsion handle, and the connector is snap-fitted and fixed to the torsion handle through a cooperation of the buckle and the slot.

8. The vascular sheath hemostatic valve according to claim 6 or 7, **characterized in that** the distal end of the connector is inserted into the proximal end of the sleeve, an annular groove is provided at a proximal opening of the sleeve, and the connector comprises an annular boss located at the distal end, and the annular boss is fitted and accommodated in the annular groove.

9. The vascular sheath hemostatic valve according to any one of claims 6 to 8, **characterized in that** a proximal end of the connector is inserted onto the torsion handle, and an end surface of the proximal end of the connector abuts against an inner side wall surface of the torsion handle;
optionally, the proximal end of the connector is inserted onto the torsion handle, and the end surface of the proximal end of the connector abuts against an inner side end surface of the torsion handle;
optionally, sealing members are provided at the joint of the torsion handle and the connector;
optionally, the sealing members are provided on the end surface of the proximal end of the connector or the inner side wall surface of the torsion handle, and/or
on an inner side wall of the proximal end of the connector or an outer side wall of the torsion handle;
optionally, the sealing members are provided on the end surface of the proximal end of the connector or the inner side end surface of the torsion handle, and/or
on the inner side wall of the proximal end of the connector or the outer side wall of the torsion handle;
optionally, the sealing members are sealing rings.

10. The vascular sheath hemostatic valve according to any one of claims 7 to 9, **characterized in that** a proximal end of the connector is inserted onto the torsion handle, an end surface of the connector abuts against the inner side wall surface of the torsion handle, and sealing rings are provided both on an abutment portion between the torsion handle and the connector, as well as on an outer side wall of the proximal end of the connector, and the proximal end of the hemostatic film tube protrudes from the proximal end of the connector and is press-fitted and fixed between the connector and the torsion handle after being folded.

11. The vascular sheath hemostatic valve according to any one of claims 1 to 10, **characterized in that** the hemostatic film tube comprises an elastic film tube, and the torsion assembly is limited and restrained on the sleeve by an axial contraction elastic force of the elastic film tube.

12. The vascular sheath hemostatic valve according to claim 11, **characterized in that** the hemostatic film tube comprises a high molecular polymer;
optionally, the high molecular polymer is selected from one or more of silicone resin, thermoplastic polyurethane elastomer, expanded polytetrafluoroethylene, polyisocyanate or rubber.

13. The vascular sheath hemostatic valve according to any one of claims 1 to 12, **characterized in that** an arc-shaped buckle is provided on an annular inner wall of the torsion handle, and a locking bevel tooth is provided on an annular outer wall of the sleeve, and the arc-shaped buckle is snap-fitted with the locking bevel tooth.

14. The vascular sheath hemostatic valve according to any one of claims 1-13, **characterized in that** a limiting boss is provided on the torsion handle, the limiting boss is protrudingly provided on an end surface of the torsion handle, and a limiting block capable of preventing the limiting boss from excessive rotation is provided on the sleeve.

15. A medical instrument, **characterized in that** the medical instrument comprises the vascular sheath hemostatic valve according to any one of claims 1 to 14 and a vascular sheath, wherein the vascular sheath comprises an inner sheath joint, the inner sheath joint is inserted onto the torsion assembly, a locking buckle is provided on the torsion assembly, and a locking groove that cooperates with the locking buckle is provided on the inner sheath joint;
optionally, the torsion assembly comprises a torsion handle and a connector arranged inside the torsion handle, the inner sheath joint is inserted onto the torsion handle, the locking buckle is provided on the torsion handle, and the locking groove that cooperates with the locking buckle is provided on the inner sheath joint.
